# EUROPEAN PATENT APPLICATION

(11) **EP 1 428 885 A1**
(43) Date of publication of application: **16.06.2004**
(21) Application number: 02762832.0
(22) Date of filing: 23.08.2002
(51) Int. Cl.: C12N 15/82, C12N 15/84, C12N 5/14

(54) **METHOD OF MODIFYING GENOME IN HIGHER PLANT**

(30) Priority: 28.08.2001 JP 2001258489
(71) Applicant: Japan Tobacco Inc., Tokyo 105-8422 (JP); Syngenta Limited, Guildford Surrey GU2 7YH (GB)
(72) Inventor: IIDA, Shigeru, Okazaki-shi, Aichi 444-0874 (JP); TERADA, Rie, Okazaki-shi, Aichi 444-0874 (JP); INAGAKI, Yoshishige, Okayama-shi, Okayama 700-0986 (JP)
(74) Representative: Reinhard - Skuhra - Weise & Partner GbR
(86) International application number: PCT/JP2002/008506
(87) International publication number: WO 2003/020940

(57) **Abstract**

A genome modification technique based on homologous recombination was established for partial modification of genomic sequences into desired sequences. The use of such a technique to achieve high efficiency and reproducibility in higher plants has been regarded as difficult. According to the present invention, endogenous genome sequences of higher plants can be targeted to efficiently and reproducibly yield homologous recombinants without changing their original loci. Moreover, the present invention enables the production of a transformed plant in which a specific gene is modified without any restrictions, and it can also contribute to the analysis of gene functions as well as to the analysis of gene expression mechanisms associated with changes in genome dynamics.

## Description

### TECHNICAL FIELD

The present invention relates to a gene construct for homologous recombination-based genome modification in higher plants, and a method for production of a higher plant having a modified genome by using a plant transformation vector carrying the gene construct. The present invention is directed to an efficient and reproducible method, in which endogenous genome sequences of plants are targeted to yield homologous recombinants without changing their original loci.

### RELATED ART

Among higher plants, gene transfer into dicotyledonous plants is often accomplished by a method based on intracellular transfer and chromosomal integration of T-DNA present in the Ti plasmid, which occur during infection with a pathogen *Agrobacterium tumefaciens*. For this purpose, a small binary vector has been developed, which is designed to have only the regions required for gene transfer from the Ti plasmid and which is capable of shuttling between *E. coli* and *Agrobacterium tumefaciens*. In monocotyledonous plants, it is common to employ direct transfer of DNA into protoplasts by electroporation or other technique, but there are also some reports of successful gene transfer by Agrobacterium infection.

However, these strategies fail to regulate the chromosomal position and copy number of introduced genes, and hence it has been difficult to express the introduced genes as expected because of various phenomena including a position effect of the introduced foreign genes, co-suppression and gene silencing.

On the other hand, there is a gene disruption method using retrotransposons, in which plant genes are disrupted by TOS17 (Applications of retrotransposons as genetic tools in plant biology. 2001 Trends in Plant Science 6:127-134). This method allows genome modification, but requires a great deal of labor to obtain a whole plant having an expectedly modified genome because retrotransposons are transpositioned to unspecified sites on the genome. Further, this method is capable of gene disruption, but incapable of mutagenesis or replacement at any site.

The T-DNA tagging method involves insertion of T-DNA into the genome via Agrobacterium infection to cause gene disruption (Plant tagnology. 1999 Trends in Plant Science 4:90-96). As in the case of using TOS17, this method also requires a great deal of labor to obtain a whole plant having an expectedly modified genome because T-DNA is inserted at unspecified sites on the genome. Further, this method is capable of gene disruption, but incapable of mutagenesis or replacement at any site.

Zhu T et al. report a technique using chimeric RNA/DNA oligonucleotides, in which chimeric RNA/DNA oligonucleotides are used to introduce single nucleotide substitutions into the genes of maize genome, thereby conferring herbicide resistance (Targeted manipulation of maize genes in vivo using chimeric RNA/DNA oligonucleotides. Proc Natl Acad Sci U S A 1999 Jul 20;96(15):8768-73). This technique is capable of inducing mutagenesis in very small regions of endogenous genes, but cannot be adapted to modification of large regions.

In view of the foregoing, a genome modification method based on homologous recombination has been developed as a potential method for expressing a foreign gene as expected. This method is characterized in that a foreign gene to be inserted is sandwiched between homologous regions to induce homologous recombination on both sides of the foreign gene. Homologous recombination refers to crossover between two homologous DNA strands.

On the other hand, the genome structure of plants contains more repetitive sequences than that of animals. In particular, it is believed that homologous recombination may be inhibited in somatic plant cells. In Arabidopsis and tobacco, a marker gene artificially inserted in the genome is targeted by homologous recombination and the frequency of homologous recombination is estimated to be 10⁻⁴ to 10⁻⁶ (Gene targeting in plants. EMBO J 1988 7:4021-4026, Extrachromosomal homologous recombination and gene targeting in plant cells after Agrobacterium mediated transformation. EMBO J 1990 9:3077-3084, Nonreciprocal homologous recombination between Agrobacterium transferred DNA and a plant chromosomal locus. Proc Natl Acad Sci USA 1993 90:7346-7350).

However, for these genome modification strategies based on homologous recombination in higher plants, there are only a few reports about the results of rudimentary experiments, as shown below. These experimental results can be divided into the following cases: i) using a genetic marker artificially inserted and ii) targeting an endogenous genome sequence of plants.

The reports as listed below are known for case i) using a genetic marker artificially inserted.

It is reported that homologous recombination of a marker gene artificially inserted in the genome of tobacco or Arabidopsis is enhanced by the expression of the *E. coli* RecA or RuvC gene or DNA double-strand cleavages by the action of the yeast HO or I-SceI endonuclease gene which is introduced to increase the frequency of homologous recombination (RecA protein stimulates homologous recombination in plants. Proc Natl Acad Sci USA 1996 93:3094-3098, Stimulation of homologous recombination in plants by expression of the bacterial resolvase RuvC. Proc Natl Acad Sci USA 1999 96:7398-7402, Enhancement of somatic intrachromosomal homologous recombination in Arabidopsis by the HO endonuclease. The Plant Cell 1996 8:2057-2066, Homologous recombination in plant cell is enhanced by in vivo induction of double strand breaks into DNA by a site-specific endonuclease. Nucleic Acid Research 1993 21:5034-5040).

Paszkowski J et al. report that genome modification by homologous recombination succeeded in tobacco protoplasts. This was the first successful case in plants, and it opened the possibility of plant genome modification by homologous recombination. The report employs a selection system in which a deleted, nonfunctional kanamycin resistance gene (positive selection marker) is first integrated into the genome and the resulting cells are then reintroduced with a gene to recover the deleted region, followed by screening for kanamycin resistance (Gene targeting in plants. EMBO J 1988 7: 4021-4026).

Risseeuw E et al. report that one line of tobacco cultured cells which homologous recombination occurred was obtained by Agrobacterium-mediated transformation. They first integrated the GUS and codA genes into the genome and then used the resulting transformed cells for retransformation with a gene that allowed partial deletion of the GUS gene and complete deletion of the codA gene upon homologous recombination so as to newly integrate the kanamycin resistance gene. They selected homologous recombinants by screening for 5-fluorocytosine sensitivity, kanamycin resistance, GUS activity and PCR, followed by Southern blotting for confirmation (Gene targeting and instability of Agrobacterium T-DNA loci in the plant genome. Plant J 1997 Apr;11(4):717-28).

Offringa R et al. report that one line of tobacco cultured cells which homologous recombination occurred was obtained by Agrobacterium-mediated transformation. This opened the possibility that Agrobacterium-mediated transformation was applicable in modification of plant genome by homologous recombination. There is no discussion about reproducibility. They also first integrated a partially deleted kanamycin resistance gene into the genome and then used the resulting cells for retransformation with a gene that complements the deleted region. They also selected homologous recombinants by screening for kanamycin resistance and PCR, followed by Southern blotting for confirmation (Extrachromosomal homologous recombination and gene targeting in plant cells after Agrobacterium mediated transformation. EMBO J 1990 Oct;9(10): 3077-84).

Halfter U et al. report that protoplasts of Arabidopsis were genetically engineered by PEG method to obtain 4 clones undergoing homologous recombination. They first integrated a partially deleted hygromycin resistance gene into the genome and then used the resulting cells for retransformation with a gene that complements the deleted region. They selected homologous recombinants by screening for hygromycin resistance and PCR, followed by Southern blotting for confirmation (Gene targeting in Arabidopsis thaliana. Mol Gen Genet 1992 Jan;231(2):186-93).

In contrast, there are only two reports shown below for case ii) targeting an endogenous genome sequence of plants to cause genome modification by homologous recombination.

Miao ZH et al. attempted to perform Agrobacterium-mediated transformation on Arabidopsis roots to obtain one line undergoing homologous recombination from 2580 cultured transformed cell lines. They inserted the kanamycin resistance gene into the TGA3 locus on the genome via homologous recombination. This was the first successful case of modifying the endogenous genome of plants by homologous recombination. In addition to screening for kanamycin resistance, they also attempted the expression of β-glucuronidase in cells into which a foreign gene had been integrated by other than homologous recombination, thereby increasing the selection efficiency. Moreover, PCR and Southern blotting were used for confirmation of homologous recombinants. However, this cell line fails to regenerate into a whole plant and hence cannot lead to the attainment of an individual which is homozygous for the homologous recombination region (Targeted disruption of the TGA3 locus in Arabidopsis thaliana. Plant J 1995 Feb; 7(2):359-65).

Yanofsky MF et al. report that Agrobacterium-mediated transformation was performed on Arabidopsis to obtain one line undergoing homologous recombination. They inserted the kanamycin resistance gene into the AGL5 gene on the genome and used kanamycin resistance and PCR for selection to obtain one line from 750 kanamycin resistant lines, followed by Southern blotting and PCR for final confirmation (Targeted disruption in Arabidopsis. Nature 1997 Oct 23;389(6653):802-3).

Also, there are reports as shown below, which indicate that homologous recombination was attempted to obtain cells undergoing homologous recombination, but such an attempt ended in failure.

Thykjaer T et al. attempted homologous recombination of Lotus japonicus using Agrobacterium. For this purpose, they used the codA gene as a negative selection marker and prepared several vectors, such as carrying this marker on one or both sides of T-DNA. However, they failed to obtain cells undergoing homologous recombination (Gene targeting approaches using positive- negative selection and large flanking regions. Plant Mol Biol 1997 Nov;35(4):523-30).

Gallego ME et al. used the kanamycin resistance gene as a positive selection marker and the codA gene as a negative selection marker in Arabidopsis transformation. However, they failed to obtain cells undergoing homologous recombination from 1 × 10⁵ transformed cells (Positive-negative selection and T-DNA stability in Arabidopsis transformation. Plant Mol Biol 1999 Jan;39(1):83-93).

### SUMMARY OF THE INVENTION

As stated above, there are various advantages in homologous recombination-based genome modification. However, for reasons such as an extremely low frequency of homologous recombination in higher plants, there are only two successful reports stated above (Miao ZH et al. and Yanofsky MF et al.) for modification of endogenous genome sequences in higher plants via homologous recombination. Neither of these reports discloses a reproducible and reliable method for homologous recombination. Moreover, no successful case is reported for monocotyledonous plants although they are very important to agricultural production. Thus, there has been a strong need to develop a reproducible and reliable method for homologous recombination in higher plants.

In view of the foregoing, the object of the present invention is to overcome various problems arising from conventional transformation of higher plant cells including monocotyledonous plant (e.g., rice) cells. Such problems include those associated with foreign gene transfer, such as the copy number and position effect of introduced foreign genes or expression instability due to co-suppression, gene silencing, etc. Thus, the present invention provides a gene construct and a vector for plant genome modification, intended for use in a method which allows transformation without causing any change in the genome other than the gene region(s) to be modified, as well as a method for producing a whole transformed plant from the transformed cells obtained by the above method.

### BRIEF DESCRIPTION OF DRAWINGS

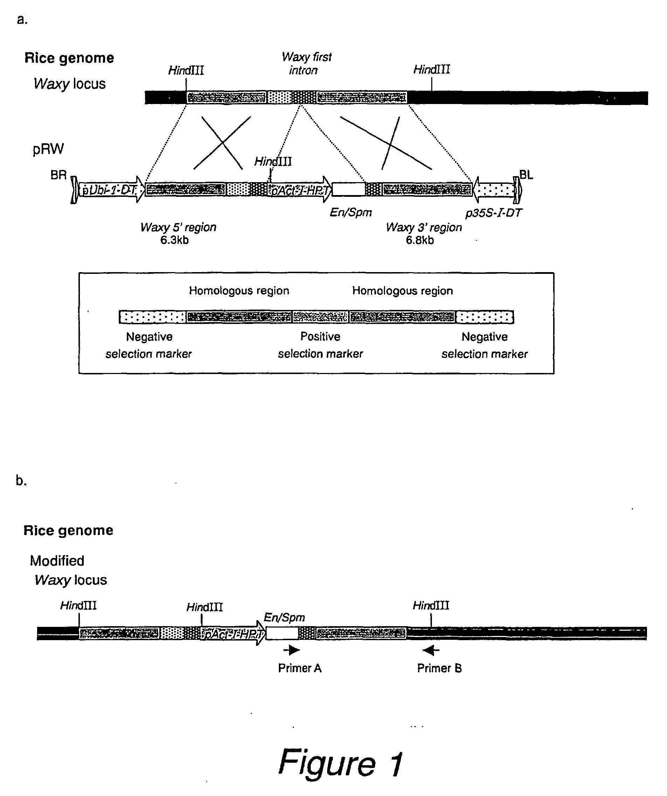

Figure 1 shows a conceptual illustration of higher plant genome modification in terms of the rice Waxy gene by way of example. Figure 1a is a schematic diagram showing homologous recombination between the introduced gene and the Waxy gene, and Figure 1b is a schematic diagram of the Waxy locus after homologous recombination.

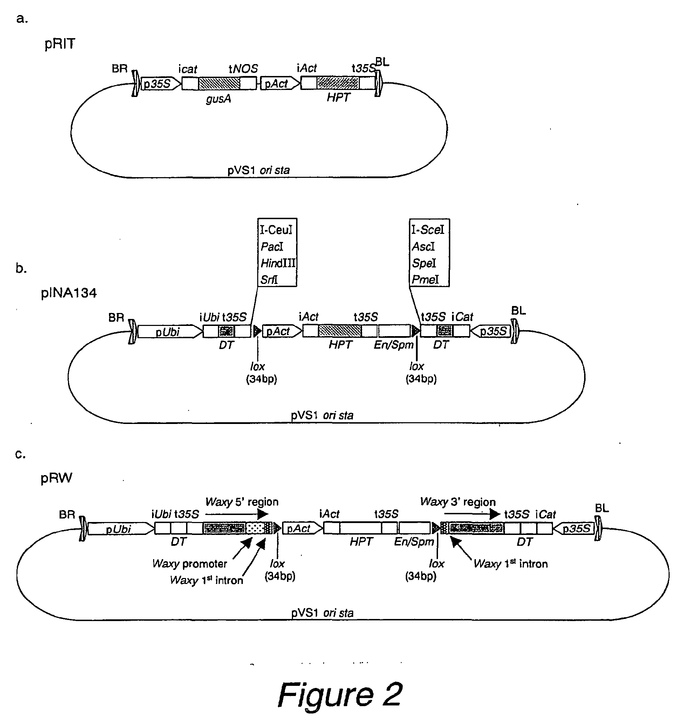

Figure 2 shows schematic diagrams of genome modification vectors. Figures 2a, 2b and 2c are schematic diagrams of a control vector, a basic vector pINA134 and a vector for Waxy gene modification, respectively. In Figure 2, the abbreviations are defined as follows: pUbi, maize ubiquitin 1 promoter; iUbi, 1st intron of maize ubiquitin 1; DT, diphtheria toxin protein A chain; lox, lox sequence of bacteriophage Cre/lox recombination system; pAct, rice actin promoter; iAct, 1st intron of rice actin; hph, hygromycin resistance gene; t35S, cauliflower mosaic virus 35S terminator; En/Spm, transcription termination region of En/Spm type transposon; iCat, 1st intron of castor bean catalase gene; p35S, cauliflower mosaic virus 35S promoter; and tNos, nopaline synthase terminator.

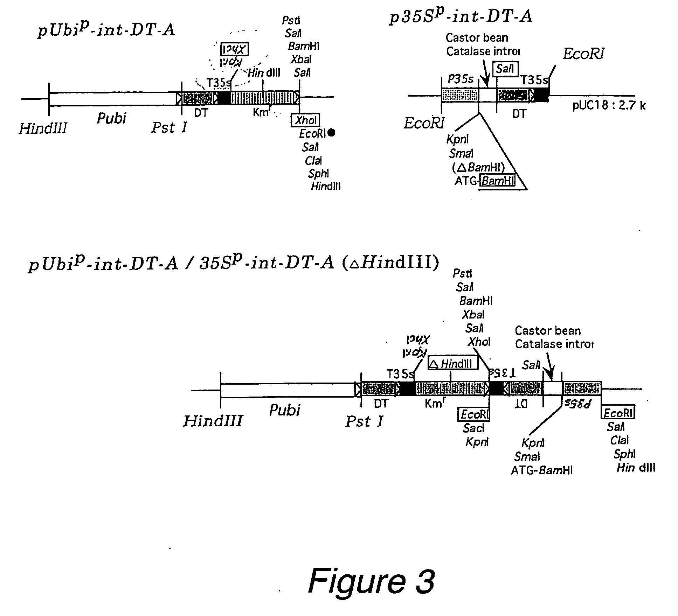

Figure 3 shows schematic diagrams of pUbi^{p}-int-DT-A, p35S^{P}-int-DT-A and pUbi^{p}-int-DT-A/35S^{P}-int-DT-A(ΔHindIII).

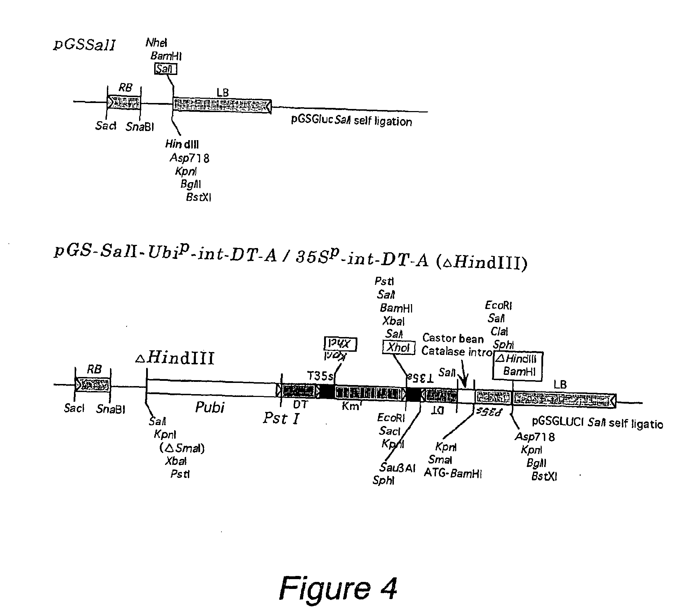

Figure 4 shows schematic diagrams of pGS-SalI and pGS-SalI-Ubi^{p}-int-DT-A/35S^{P}-int-DT-A(ΔHindIII).

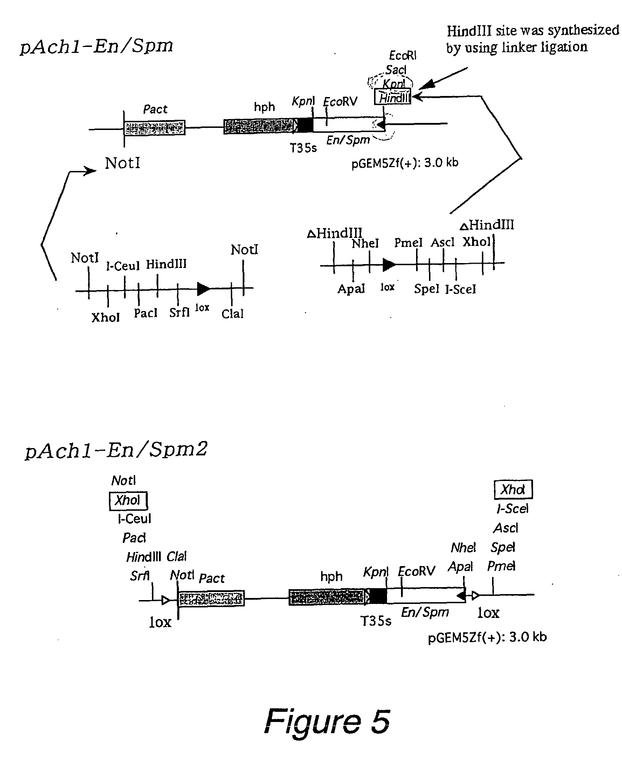

Figure 5 schematically shows the construction of vector pINA134, i.e., schematic diagrams of pAch1-En/Spm and pAch1-En/Spm2.

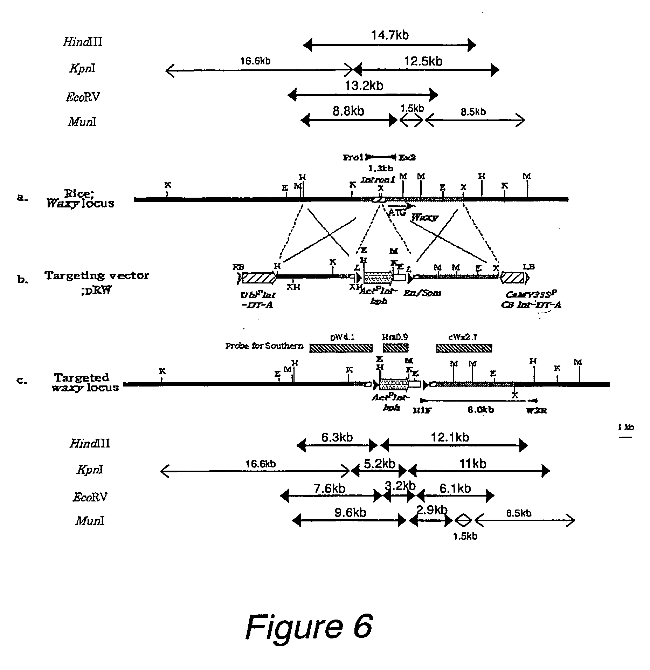

Figure 6 schematically shows physical maps of the Waxy locus and a targeting vector, as well as a physical map after homologous recombination.

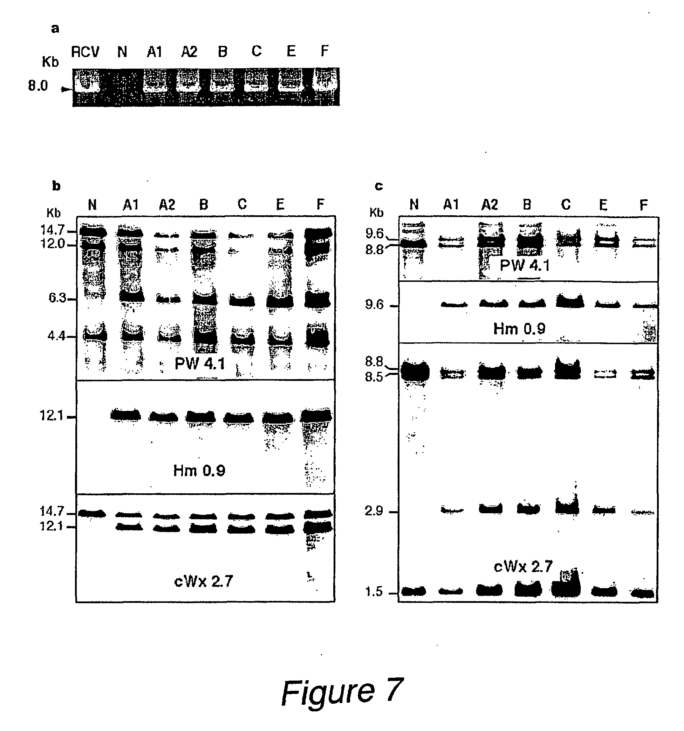

Figure 7 shows analyses of homologous recombinants. Figure 7a shows PCR analysis using HIF and W2R primers. In addition to this, Figure 7 shows the results of Southern analysis in which DNA was isolated from green leaves of whole plants regenerated from PCR-positive clones and then digested with HindIII or MunI. Figure 7b shows genomic Southern analysis using HindIII. Figure 7c shows genomic Southern analysis using MunI. In Figure 7, RCV denotes a positive control vector and N denotes a non-recombinant, while A1, A2, B, C, E and F denote whole plants regenerated from lines A-I, A-II, B, C, E and F, respectively.

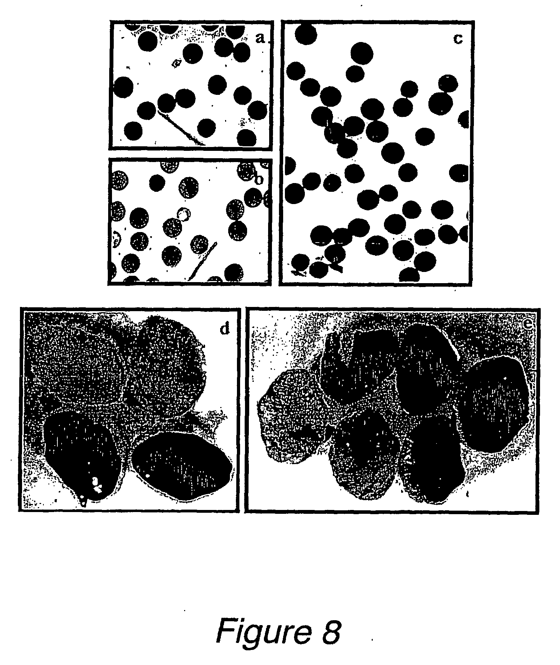

Figure 8 shows the results of iodine staining.

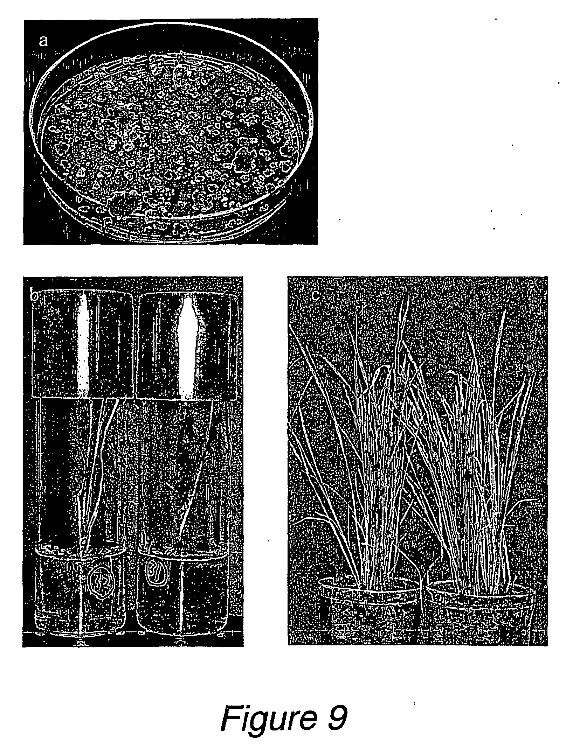

Figure 9 shows the appearance of homologous recombinants during regeneration. Figure 9a shows hygromycin-resistant calli, Figure 9b shows regenerated rice plants, and Figure 9c shows regenerated rice plants after the earing stage.

### DETAILED DESCRIPTION OF THE INVENTION

The present invention provides a gene construct for modifying the genome of higher plants by homologous recombination, which contains the following elements between BR (right border sequence) and BL (left border sequence) of T-DNA in the following orientation from BR:
(1) a first negative selection marker gene in an expressible state, preferably in a highly expressible state;
(2) a first cloning site for incorporating the 5' region of a gene to be homologously recombined with a target gene in the host genome;
(3) a positive selection marker gene in an expressible state, preferably in a highly expressible state;
(4) a second cloning site for incorporating the 3' region of the gene to be homologously recombined with the target gene in the host genome; and
(5) a second negative selection marker gene in an expressible state, preferably in a highly expressible state, which may be the same as the first negative selection marker. The present invention also provides a plant transformation vector comprising the gene construct stated above.

The gene construct of the present invention is designed to have, between BR and BL of T-DNA, expressible negative selection markers (preferably in a highly expressible state) located on the 5' upstream and 3' downstream sides of an expressible positive selection marker (preferably in a highly expressible state), and to have cloning sites (preferably multiple cloning sites) between the positive selection marker and the negative selection markers in order to incorporate sequences to be homologously recombined. This construct is further integrated into a vector for Agrobacterium-mediated plant transformation and the resulting vector is called a "basic vector" in the present invention (Figure 2b).

T-DNA is a part of an endogenous Ti plasmid in Agrobacterium. When plants are infected with Agrobacterium, the bacterial cells per se fail to enter plant cells, but the T-DNA region as a part of the Ti plasmid present in the bacterial cells is transferred into plant cells. T-DNA is delimited by BR (right border sequence) and BL (left border sequence). Each border sequence consists of about 25 bp incomplete repeats.

The gene construct of the present invention contains negative and positive selection markers. This is because higher plants have a very high frequency of transformation mediated by non-homologous recombination and hence it is necessary to efficiently select homologous recombinants from non-homologous recombinants. To this end, these markers are used for negative and positive selections.

Positive selection is a technique for selecting transformed cells after gene transfer under conditions allowing the survival of only cells expressing the gene product. For example, if the neomycin resistance gene is introduced, the gene-introduced cells are selected based on their resistance to G418, an analog of neomycin.

Negative selection is a technique for selecting transformed cells after gene transfer under conditions causing the death of cells expressing the gene product. For example, if cells are introduced with the thymidine kinase gene of human herpes virus and screened in the presence of ganciclovir (an analog of thymidine), cells expressing the thymidine kinase gene are killed.

In the present invention, positive-negative selection (a combination of positive and negative selections) is used as a selection procedure. This selection procedure involves first selecting transformants with the aid of a positive selection marker, and then selectively picking up homologous recombinants to remove non-homologous recombinants on the basis of the fact that a negative selection marker causes the death of transformants produced by other than homologous recombination. This procedure achieves increased selection efficiency.

A negative selection marker should be toxic to plant cells, but non-toxic to *E. coli* and Agrobacterium used for vector manipulation. Such a marker is preferably the diphtheria toxin protein A chain gene. In addition to this, examples of a gene available for use as a negative selection marker include the codA gene, the cytochrome P-450 gene and the barnase gene.

The following reports are known about negative selection markers. Koprek T. et al. report that the codA gene and bacterial P-450 can be used as negative selection markers for barley (Negative selection systems for transgenic barley (Hordeum vulgare L.): comparison of bacterial codA - and cytochrome P450 gene-mediated selection. Plant J 1999 Sep;19(6):719-26). Terada et al. report that the diphtheria toxin protein gene (DxT-A) can be used as a negative selection marker for rice (Negative selection effects of the diphtheria toxin protein (DxT-A) gene in rice and its application to gene targeting. 22nd Annual Meeting of the Molecular Biology Society of Japan, 1999 Dec;:2P-0343).

Preferred examples of a positive selection marker include the hygromycin resistance gene, as well as the kanamycin resistance gene, the imidazoline resistance gene, the glyphosate resistance gene, the phosphomannose-isomerase gene, the blasticidin S resistance gene, the bar gene and the glyphosinate resistance gene.

A feature of the present invention is to ensure the expression (preferably high-level expression) of positive and negative selection markers for the purpose of efficient selection of cells undergoing homologous recombination in higher plants having a low frequency of homologous recombination. As shown in Figures 2b and 2c, in order to enhance the expression of negative selection markers, a promoter (e.g., cauliflower mosaic virus 35S promoter) may be ligated to an intron to achieve enhanced expression, or alternatively, a ubiquitin promoter capable of driving strong expression in monocotyledonous plants may be used. Likewise, it is also desirable to enhance the expression of a positive selection marker. To this end, when the hygromycin resistance gene is used as a positive selection marker, it is desirable to use an actin promoter capable of driving strong expression of the hygromycin resistance gene. To ensure high-level expression of these selection markers, other elements may also be used, including a maize ubiquitin promoter, a rice PLD intron and a castor bean catalase intron.

Another feature of the present invention is to use a negative selection marker gene on each of the BL and BR sides. They may be different genes, but are preferably the same. The inventors of the present invention have analyzed the reason why genome modification by homologous recombination ended in failure in higher plants, particularly in monocotyledonous plants, and they have reached a conclusion that there are a considerable number of non-homologous recombinants having a negative selection marker gene(s) integrated in an incomplete form, leading to low efficiency of negative selection. In the present invention, the use of two negative selection markers located apart from each other sufficiently reduces the probability that non-homologous recombinants will survive selection steps.

The gene construct of the present invention contains two cloning sites, one being located between a positive selection marker gene and a first negative selection marker gene, and the other being located between the positive selection marker gene and a second negative selection marker gene. Any cloning site may be used as long as it is suitable for incorporating the 5' or 3' region of a gene to be homologously recombined with a target gene. A multiple cloning site is preferred for use as a cloning site because it is available for incorporation of any gene. A multiple cloning site may be prepared by flexibly designing and synthesizing an oligo DNA sequence having desired restriction enzyme sites.

The 5' region of a gene to be homologously recombined is incorporated into the first cloning site, while the 3' region of the gene is incorporated into the second cloning site.

A gene to be homologously recombined may be a structural gene encoding a specific protein or may be other gene affecting plant phenotypes. These genes may be used to render a target gene in the plant genome functionally deficient or may be used to modify a target gene to give a modified recombinant plant. Non-limiting examples of a target gene include the Waxy gene and the EPSPS gene.

There is no particular limitation on how to select the border between "5' region" and "3' region" of a gene to be homologously recombined. For example, in an attempt to block the functions of a target gene, a gene to be homologously recombined may be almost halved or divided in a ratio of 7:3 to 3:7. The resulting upstream and downstream parts may be used as "5' region" and "3' region," respectively, but in general, each region preferably has a longer length. To ensure homologous recombination, the length of each region is preferably longer than at least 1000 bases and is set to at least 500 bases.

In contrast, in an attempt to modify the functions of a target gene, a gene to be homologously recombined is divided into "5' region" and "3' region" and inserted into the respective cloning sites. If a gene to be homologously recombined has an appropriate intron, the gene can be divided within the intron into 5' and 3' regions. Further, it should be understood that the "5' region" may be a sequence having all functions required for expression of a gene to be homologously recombined, while its downstream flanking region may be used as the "3' region." Alternatively, the "3' region" may be a sequence having all functions of a gene to be homologously recombined, while its upstream flanking region may be used as the "5' region."

In order that the positive selection marker can be excised in the progeny of transformed plants using a site-specific recombination system, the gene construct may further be designed to have recombinase recognition sequences as element (7) (e.g., lox sequences of the Cre/lox recombination system, RS sequences of the R/RS recombination system, or FRT sequences of the FLP-FRT recombination system) in upstream and downstream regions of the positive selection marker gene (Figures 2b and 2c). The Cre/lox recombination system involves the use of Cre recombinase in combination with lox to excise unwanted parts. The R/RS recombination system involves the use of recombinase encoded by the R gene in combination with RS sequences to induce excision of a DNA region sandwiched between two RS sequences. Likewise, the FLP-FRT recombination system involves the use of FLP recombinase in combination with FRT sequences to induce excision of a DNA region sandwiched between two FRT sequences.

For example, when Cre recombinase is expressed after homologous recombination using the gene construct of the present invention designed to have the Cre/lox recombination system, it is possible to excise a sequence containing the positive selection marker gene, which is sandwiched between two lox sequences in the homologous recombination region of the genome. After expression of Cre recombinase to excise a region sandwiched between two lox sequences in the genome, the 5' and 3' regions of the gene introduced into the plant genome via homologous recombination can be linked in the original form as found in nature. The excision can be confirmed by PCR or Southern blotting.

In order that Cre recombinase is expressed in a callus to excise a region between lox sequences, the excision may be accomplished by crossing with another plant having the introduced Cre recombinase gene or by introducing the Cre recombinase gene into a homologously recombined plant.

The combination of Cre and lox may be used for modification as well as deletion of a sequence between lox sites. For modification purposes, a homologous recombinant is crossed with a recombinant plant having the introduced Cre recombinase gene to excise a sequence between lox sites, and then crossed with another recombinant having Cre recombinase and a gene to be integrated between lox sites, whereby the gene to be integrated is inserted into the lox sequence site remaining after excision. Examples of a modification include, but are not limited to, insertion of a herbicide resistance gene. Alternatively, in an attempt to regulate the expression of a target gene, the gene construct may also be designed to have a transcription termination region, preferably a transcription termination region of En/Spm type transposon, as element (6) to ensure inhibition effects (Figures 2b and 2c).

Using standard genetic engineering techniques, those skilled in the art will readily ligate the above elements (1) to (5) as well as elements (6) and/or (7), if present, in the predetermined order to give the gene construct of the present invention. Further, when using standard genetic engineering techniques, it is also easy for those skilled in the art to choose a gene to be homologously recombined with a target gene in the plant genome, and to insert the 5' and 3' regions of the gene into the first and second cloning sites, respectively.

The gene construct of the present invention is ligated to a vector for Agrobacterium-mediated plant transformation at an appropriate restriction enzyme site such that the gene construct replaces the T-DNA region or the region between BR and BL of the vector. The resulting vector corresponds to the basic vector as used herein. The gene modification vector of the present invention can be derived from this basic vector when the 5' and 3' regions of a gene to be homologously recombined are integrated into the first and second cloning sites of the basic vector, respectively.

For example, the rice Waxy gene and its flanking regions may be introduced into the basic vector to construct a vector for rice Waxy gene modification (Figure 2c).

The present invention also provides a method for producing a higher plant having a modified genome by means of homologous recombination, comprising the following steps:
(a) introducing the gene modification vector of the present invention into an Agrobacterium strain which contains Ti plasmid;
(b) allowing said Agrobacterium strain to infect a cell, tissue or callus of a plant;
(c) performing negative and positive selections to select a cell, tissue or callus undergoing homologous recombination;
(d) culturing the cell or tissue to form a callus in the case that selection was performed with a cell or tissue;
(e) culturing the callus in a regeneration medium to generate an individual plant which is heterozygous for homologous recombination in the genome; and
(f) fertilizing said plant to yield a gene-modified higher plant which is homozygous for homologous recombination.

Since higher plants have a very low frequency of homologous recombination, as stated above, the above steps (a) and (b) are desirably accomplished by high-frequency Agrobacterium- mediated transformation. The gene modification vector may be introduced into, e.g., rice callus by high-frequency Agrobacterium-mediated transformation, followed by application of positive-negative selection to select potential callus undergoing homologous recombination. High-frequency Agrobacterium-mediated transformation was proposed by Terada et al. as an improvement on Agrobacterium-mediated gene transfer to rice, etc. This transformation procedure achieves 5-fold to 10-fold higher efficiency of gene transfer than conventional procedures (Development of Agrobacterium-mediated rice transformation with a high level of transformation efficiency, 96th Lecture Meeting of Japanese Society of Breeding, 1999 Sep;:212). This transformation is based on a binary vector system and is characterized by using the hygromycin gene in a highly expressible state.

Positive-negative selection may be performed as follows. The callus cultures from step (d) are transferred to a positive selection medium. Such a positive selection medium may be a normal medium for callus culture supplemented with components required for positive selection.

In a case where antibiotic resistance is used for positive selection, the medium is further supplemented with the corresponding antibiotic in such a concentration that calli untransformed with the selection marker gene cannot survive or are at least unable to grow. In the case of using hygromycin as an antibiotic, hygromycin is added in an amount of 10 to 200 mg per liter of medium.

The calli are grown in the dark for 2 to 3 weeks under conditions of 15°C to 40°C. Each surviving callus is selected as a candidate successfully undergoing homologous recombination, which has the positive selection marker gene and no negative selection marker gene. Differences in color and proliferation of callus may be used to determine whether a callus survives on the medium.

Further, PCR analysis may be performed to confirm that these candidates carry the intended gene modification.

In PCR analysis of DNA extracted from callus cells, primers are designed such that one of the primers binds to the region of the inserted gene, while the other binds to a partial sequence of any gene beyond the border of the inserted fragment. PCR fails to produce fragment amplification in cells undergoing no gene insertion event or undergoing non-homologous recombination because the two primers are not located in proximity to each other. In contrast, PCR gives an amplified fragment of the expected size in cells undergoing homologous recombination because both primers bind to regions located close to each other. The detection of this fragment indicates a high possibility that the corresponding cell population contains the homologously recombined gene. Such a cell population is divided into subpopulations and tested again by PCR. This cycle may be repeated to isolate cells undergoing homologous recombination.

Instead of or subsequent to the PCR analysis stated above, a detailed analysis may be performed by Southern blotting to confirm gene modification.

The method of the present invention enables high-efficiency homologous recombination in monocotyledonous plants such as rice. As shown in the examples below, in a preferred embodiment, six experiments each produce more than 100 callus on average from 400 seeds after positive-negative selection, and at least one gene-modified plant is obtained from these callus in every experiment. In view of these facts, the method of the present invention is highly reproducible and reliable.

Each callus confirmed to have an expectedly modified gene may be grown in a regeneration medium such as MS medium to give an individual plant which is heterozygous for homologous recombination in the genome. The structure and expression of the modified gene in pollen and seeds are then analyzed by PCR and/or Southern blotting, as in the case above, to confirm whether homologous recombination occurs heterozygously at the target locus.

Since the resulting individuals are capable of regeneration and fertilization, they may be self-pollinated to yield gene-modified higher plants which is homozygous for homologous recombination.

### ADVANTAGES OF THE INVENTION

In view of the foregoing, according to the present invention, even in higher plants which have a low frequency of homologous recombination, but a very high frequency of gene transfer mediated by non-homologous recombination, endogenous genome sequences of such plants can be targeted to reproducibly yield one homologous recombinant in every 100 resulting callus without changing their original loci when negative selection markers are ligated to both sides of the homologous region and a positive selection marker is also used to perform positive-negative selection.

Moreover, the present invention enables the production of a transformed plant in which a specific gene is modified without any restrictions. For example, in a case where the method of the present invention is used to modify the rice Waxy gene involved in starch synthesis, it is possible to yield a rice plant capable of regulating Waxy gene expression. Such a gene-modified rice plant also has a possibility of further genome modification mediated by site-specific recombination because sequences required for site-specific recombination are integrated at the same locus. The present invention can also contribute to the analysis of gene functions as well as to the analysis of gene expression mechanisms associated with changes in genome dynamics.

In addition, it is possible to regenerate a homologous recombinant into a whole plant. The resulting plant is capable of fertilization, and hence it is also possible to yield an individual plant which is homozygous for the homologously recombined genome.

### EXAMPLES

### Example 1: Control vector and Waxy targeting vector

Vectors pRIT (Figure 2a), pINA134 (Figure 2b) and pRW (Figure 2c) were constructed using the rep and sta regions of the pVS1-derived pGSGluc vector (Saalbach I, Pickardt T, Machemehl F, Saalbach G, Schieder O, Müntz K (1994) A chimeric gene encoding the methionine-rich 2S albumin of the Brazil nut (Betholletia excelsa H.B.K.) is stably expressed and inherited in transgenic grain legumes. Mol Gen Genet 242: 226-236) as well as the spectinomycin resistance gene.

### Example 1-1

### Construction of pRIT

pGSGluc was digested with SalI to give a fragment of approximately 9.4 kbp, which was then self-ligated to construct pGS-SalI. pGS-SalI was digested with HindIII and ligated to HindIII-digested pIG221 (Tanaka A, Mita S, Ohta S, Kyozuka J, Shimamoto K, Nakamura K (1990) Enhancement of foreign gene expression by a dicot intron in rice but not in tobacco is correlated with an increased level of mRNA and an efficient splicing of the intron. Nucleic Acids Res. 18:6767-6770), followed by digestion with EcoRI and BglII to give a 12.4 kbp fragment. This fragment was self-ligated using a synthetic linker having EcoRI, NotI and BglII sites to yield pGSIG. A 2.6 kbp fragment containing a rice Actin 1 promoter, the first intron thereof, the hph gene and a CaMV35S terminator was excised from pAch1 (Bilang R, Iida S, Peterhans A, Potrykus I, Paszkowski J (1991) The 3'-terminal region of the hygromycin-B-resistance gene is important for its activity in Escherichia coli and Nicotiana tabacum. Gene 100:247-250) and then inserted into the EcoRI site of pGSIG to construct pRIT having, between right and left borders, the hph gene under control of the rice Actin 1 promoter with the first intron (Act^{p}-int-hph gene) and the gusA gene under control of CaMV35S.

### Example 1- 2

### Construction of pINA134

The diphtheria toxin A chain gene (DT-A gene) was obtained as a fragment by PCR using pMC1DpA as a template (Yagi T, Ikawa Y, Yoshida K, Shigetani Y, Takeda N, Mabuchi I, Yamamoto T, Aizawa S (1990) Homologous recombination at c-fyn locus of mouse embryonic stem cells with use of diphtheria toxin A-fragment gene in negative selection. Proc. Natl. Acad. Sci. USA 87:9918-9922), and then used to replace the coding region of the hph gene in pCKR138 (Izawa T, Miyazaki C, Yamamoto M, Terada R, Iida S, and Shimamoto K (1991) Introduction and transposition of the maize transposable element Ac in rice (Oriza sativa L.). Mol Gen Genet 227:391-396) to allow the ligation of a CaMV35S promoter, DT-A and a CaMV35S terminator, thereby constructing plasmid p35S^{P}-DT-A. Castor bean catalase (Ohta S, Mita S, Hattori T, Nakamura K (1990) Construction and expression in tobacco of a β-glucuronidase (GUS) reporter gene containing an intron within the coding sequence. Plant Cell Physiol 31:805-813) was inserted between the CaMV35S promoter and the DT-A gene to construct p35S^{P}-int-DT-A. The fragment to be inserted was obtained by PCR. The CaMV35S promoter of p35S^{P}-DT-A was replaced with a 2.0 kb fragment containing a maize Ubiquitin 1 promoter and the first intron thereof, which had been obtained by PstI digestion of pAHC27 (Takimoto I, Chritstensen AH, Quail PH, Uchimiya H, Toki S (1994) Non-systemic expression of a stress-responsive maize polyubiquitin gene (Ubi-1) in transgenic rice plants. Plant Mol Biol 26: 1007-1012), to construct plasmid pUbi^{p}-int-DT-A. The kanamycin resistance gene derived from Tn930 (Grindley NDF and Joyce CM (1981) Analysis of the structure and function of the kanamycin-resistance transposon Tn903. Cold Spring Harb. Symp. Quant. Biol. 45:125-133) was inserted at the 3'-end of the CaMV35S terminator in pUbi^{p}-int-DT-A in the same orientation as DT-A, followed by inserting a fragment covering from the promoter to the terminator of p35S^{P}-int-DT-A at the 3'-end of the kanamycin resistance gene in the reverse orientation to construct pUbi^{p}-int-DT-A/35S^{P}-int-DT-A. pUbi^{p}-int-DT-A/35S^{P}-int-DT-A was digested with HindIII, blunt-ended and self-ligated to remove the HindIII site present in the kanamycin resistance gene, thereby constructing pUbi^{p}-int-DT-A/35S^{P}-int-DT-A (ΔHindIII). pUbi^{p}-int-DT-A/35S^{P}-int-DT-A (ΔHindIII) was then cleaved with HindIII to give the region sandwiched between Ubiquitin 1 promoter and CaMV35S promoter, which was then inserted into the HindIII site of pGS-SalI to construct pGS-SalI-Ubi^{p}-int-DT-A/35S^{P}-int- DT-A. The resulting construct was partially digested with HindIII and inserted with a linker (5'- AGC TGG GAT CCC -3') to remove both of two HindIII sites, thereby constructing pGS-SalI-Ubi^{p}-int-DT-A/35S^{P}-int-DT-A (ΔHindIII).

A transcription termination region of maize En/Spm transposon (Gierl A, Schwarz-Sommer Z, and Saedler H (1985) Molecular interactions between the components of the En-I transposable element system of Zea mays. EMBO J. 4:579-583) was synthesized by PCR as a 1.0 kb fragment and then inserted at the 3'-end of the CaMV35S terminator in pAch1 to construct pAchl-En/Spm. PCR was also carried out to synthesize fragments, each carrying the loxP sequence of the Cre/loxP sit-specific recombination system derived from bacteriophage P1 (Kanegae Y, Lee G, Sato Y, Tanaka M, Nakai M, Sakaki T, Sugano S and Saito I (1995) Efficient gene activation in mammalian cells by using recombinant adenovirus expressing site-specific Cre recombinase. Nucl Acids Res 23:3816-3821) and a cloning site used for insertion of a homologous sequence in constructing a targeting vector. These synthesized fragments were inserted at the 5'- and 3'-ends of the Act^{p}-int-hph-En/Spm gene in pAch1-En/Spm to construct pAch1-En/Spm2. The resulting construct was used to replace the region of the kanamycin resistance gene in pGS-SalI-Ubi^{p}-int-DT-A/ 35S^{P}-int-DT-A (ΔHindIII) to construct pINA134.

### Example 1-3

### Construction of pRW

A BAC clone (123-D4) containing the Waxy gene and its flanking region (67 kb) of a japonica rice variety "Shimokita" was digested with HindIII and the resulting 14.7 kb fragment containing the Waxy gene was inserted into the HindIII site of pBC (Stratagene Co.). After digestion with XbaI, the resulting 6.3 kb and 6.8 kb fragments were respectively inserted into the XbaI site of pBluescript SK-(Stratagene Co.) to construct pBluescrip-Waxy 6.3kb and pBluescrip-Waxy 6.8kb. An AscI linker was inserted into the NotI site present in the 3'-terminal multiple cloning site of pBluescrip-Waxy 6.8kb, followed by digestion with SmaI and AscI to give a 6.8 kb fragment. This 6.8 kb fragment was inserted between the PmeI and AscI sties of pINA134 to construct pINA-Waxy 6.8kb. The pBluescript carrying the 6.3 kb fragment containing the promoter region of the Waxy gene was digested with HindIII and the resulting 6.3 kb fragment was inserted into the HindIII site of pINA-Waxy 6.8kb to construct a Waxy targeting vector pRW.

These vectors were respectively introduced into *Agrobacterium tumefaciens* strain EHA101 (Hood EE, Fraley RT, Chilton M-D (1986) The hyper-virulence of Agrobacterium tumefaciens strain A281 on legumes. Plant Physiol. 83:529-534) by electroporation (Sambook, J, Fritsch, EF, Maniatis, T. (1989) Molecular Cloning: A Laboratory, manual, 2nd Edn. Cold Spring Harbor, NY: Cold Spring Harbor Laboratory Press).

### Example 2: Transformation

Agrobacterium-mediated transformation was performed primarily according to the method of Hiei et al. (Hiei Y, Ohta S, Komari T, Kumashiro T (1994) Efficient transformation of rice (Oryza sativa L.) mediated by Agrobacterium and sequence analysis of the boundaries of the T-DNA. The Plant Journal 6:271-282), provided that Agarose Type I (Sigma Co.) was used for rice cell culture, instead of Gelrite. Mature seeds of a rice variety Nipponbare were used for introduction of callus. After removing husks, the seeds were soaked in 70% ethanol for 1 minute and then sterilized by soaking in 2.5% sodium hypochlorite for 20 minutes. The sterilized seeds were then rinsed with sterile water, transferred to 2N6 medium using Agarose Type I and grown in the dark at 28°C for 3 to 4 weeks. They were further transferred to another 2N6 medium and grown for an additional 3 to 7 days.

Agrobacterium strain EHA101 carrying pRIT, pINA134 or pRW was cultured at 28°C for 3 days on AB medium supplemented with 125 mg/l spectinomycin (Chilton MD, Currier TC, Farrand SK, Bendich AJ, Gordon MP, Nester EW (1974) Agrobacterium tumefaciens DNA and PS8 bacteriophage DNA not detected in crown gall tumors. Proc Natl Acad Sci U S A 71:3672-3676). After culture, the cells were collected and suspended in AAM solution supplemented with 400 µM acetosyringone (Hiei Y, Ohta S, Komari T, Kumashiro T (1994) Efficient transformation of rice (Oryza sativa L.) mediated by Agrobacterium and sequence analysis of the boundaries of the T-DNA. The Plant Journal 6:271-282), followed by shaking at 100 rpm for 1 to 2 hours at 28°C. Each callus was soaked for 3 to 4 minutes in a solution having a bacterial concentration adjusted to OD₆₀₀ = 0.18. After removing excess bacterial cells with a sterilized paper towel, each callus was transferred to 0.8% Agarose Type I-containing 2N6-AS medium supplemented with 400 µM acetosyringone and grown in the dark at 28°C for 3 days. Each callus was washed with sterile water containing 500 mg/l cefotaxime. After removing excess water with a sterilized paper towel, each callus was transferred to 0.8% Agarose Type I-containing 2N6-CH medium supplemented with 50 mg/l hygromycin and 500 mg/l cefotaxime, and then grown in the dart at 28°C for 3 to 4 weeks. After counting callus growing continuously, individual callus were transferred to multi-well plates containing 2N6-CH solution and provided for subsequent analysis.

To examine the transformation efficiency of positive-negative selection in transformation with pRW and the number of "escape" calli (resistant to hygromycin, but undergoing no homologous recombination), the number of callus obtained by transformation with pRIT or pINA134 was compared to that of pRW. This comparison was accomplished based on the fresh weight of uninfected callus and the number of resulting callus. In each experiment, about 40 gFW (gram fresh weight) of healthy callus was obtained from 400 mature seeds. One to five grams fresh weight of callus was used for transformation with pRIT and pINA134, and the rest was used for pRW. After selection on 2N6-CH, hygromycin-resistant calli were counted.

Callus redifferentiation was induced using MSRE medium containing MS salt (Murashige, T. and Skoog. F. (1962) A revised medium for rapid growth and bioassays with tobacco tissue cultures. Plant Physiol. 15, 473), 30 g/l sucrose, 30 g/l sorbitol, 1 mg/l NAA, 2 mg/l BAP, 50 mg/l hygromycin, 500 mg/l cefotaxime and 0.8% Agarose Type I. Each redifferentiated plant was grown on MS medium containing 30 g/l sucrose, 50 mg/l hygromycin, 500 mg/l cefotaxime and 0.8% Agarose Type I, and then transferred to a greenhouse.

### Example 3: Detection of targeted callus

Each callus (30 mgFW) was introduced into a 2 ml plastic tube and crushed with a FastPrep tissue crusher (FP120; Bio 101 Co.) at a speed of 6.5 for 20 seconds in the presence of 0.5 ml CTAB solution (Plant DNA isolation kit. Kurabo Co.) and 5 ceramic balls (diameter: 3 mm; Nikkato Co.). The crushed product was incubated at 65°C for 0.5 to 2 hours and then treated in an automated DNA extractor (NA2000. Kurabo Co.) to extract and purify its DNA. In the case of using leaves, 10 mgFW of leaves in a tube was frozen in liquid nitrogen and crushed roughly, followed by additional crushing with a FastPrep tissue crusher at a speed of 6.5 for 10 seconds. Subsequently, the same procedure as used for callus was repeated to extract DNA. The resulting DNA was dissolved in 10 mM Tris-EDTA buffer (pH 8.0) and assayed for absorbance at 260 nm. PCR was carried out using LA Taq (Takara Co.). The reaction condition was 1 cycle of 94°C for 1 minute, 32 cycles of 94°C for 1 minute and 60°C for 20 minutes, and then 1 cycle of 72°C for 10 minutes. Homologous recombination was confirmed using primers H1F: 5'- GTA TAA TGT ATG CTA TAG GAA GTT ATG TTT -3' and W2R: 5'- GTT TGG TCA TAT TAT GTA CTT AAG CTA AGT -3'. The original promoter of the Waxy gene was confirmed using Pro1: 5'- ACA CAA ATA ACT GCA GTC TC -3' and Ex2: GAG CTG GGA CGT GGT GAG AGC CGA CAT G -3'. The amplified DNA fragments were electrophoresed on a 0.6% agarose gel and visualized with ethidium bromide.

### Example 4: Iodine staining of albumen and pollen

Endosperm was cut with a razor knife. Anthers were collected immediately before dehiscence and treated with a fixing solution (acetic acid:ethanol = 1:3 v/v) before staining. Endosperm and pollen were stained with iodine solution (0.18% (w/v) iodine, 1% (w/v) potassium iodide).

### Example 5

### a. Southern blot analysis

The DNAs of Nipponbare and transformants were extracted from their green leaves by the CTAB method and then purified (Murray MG, Thompson WF. (1980) Rapid isolation of high molecular weight plant DNA. Nucleic Acids Res 8:4321-4325). The plasmid DNA of the BAC clone 123-D4 was isolated with a nucleic acid extraction kit (Qiaprep Spin Maxiprep Kit. Qiagen Co.). The isolated DNAs were digested with HindIII, KpnI, MunI or EcoRV, separated on a 0.7% agarose gel, and then transferred to a Hybond N membrane (Amersham Co.). A 2.7 kbp fragment obtained by EcoRI digestion of plasmid Adh1-antiWx was used as a cWx2.7 probe. The cWx2.7 probe contains the Waxy coding region (2.3 kb) and the Waxy first intron (about 400 bp). A 4.1 kbp fragment obtained by XhoI digestion of plasmid pBluescrip-Waxy 6.3kb was used as a pWx4.1 probe. These fragments were labeled with digoxigenin (Boehringer Mannheim Biochemica). A labeled probe for the hph gene was prepared with a PCR DIG probe synthesis Kit (Roche Co.) using primers F1Hm: 5'- GTA GGT AGA CCG GGG GCA ATG AG -3' and R2Hm: 5'- ACG CCC GAC AGT CCC GGC TCC GG -3'. The PCR condition was 1 cycle of 94°C for 2 minutes, 38 cycles of 94°C for 30 seconds, 60°C for 45 seconds and 68°C for 45 seconds, and then 1 cycle of 72°C for 3 minutes. The DNA fragments transferred on each membrane were hybridized at 42°C with the labeled fragments in a solution containing 50% formamide, 5 × SSC, 2% blocking solution (Boehringer Mannheim Biochemica), 0.2% SDS and 0.1% lauryl sarcosine. The hybridized probes were detected by CDP-Star (Boehringer Mannheim) and anti-digoxigenin alkaline phosphatase (Boehringer Mannheim).

### b. Nucleotide sequencing of recombination region

The DNA isolated from green leaves of each transformant was used as a template to carry out PCR using DNA polymerase, AmpliTaq Gold (Perkin Elmer). The reaction condition was 1 cycle of 94°C for 10 minutes, 36 cycles of 94°C for 0.5 minutes, 55°C for 0.5 minutes and 72°C for 1 minute, and then 1 cycle of 72°C for 10 minutes.

The following 12 pairs of primers were used for the reactions: WxR-F1: 5'- AAG CTT CTA TTG ATG CAT AC -3'and WxR-R1391: 5'- GAC AGG ACA AGAG CTG AGG GGA AC -3'; WxR-F1276: 5'- TAT AAT GGC CCA GAG TAG TA -3' and WxR-R1788: 5'- TTG CCC AGT TGC CCA AAG AA -3'; WxR-F1713: 5'- CTC TCC CCT CCT CTC CCT TTC T -3' and WxR-R2396: 5'- TCC TTG CAT TAT GTG ATG GA -3'; WxR-F2363: 5'- CCA ATC CAA TCC AAT CCA TCA C -3'and WxR-R2925: 5'- CCC TCT TCT CTC CCT TTT GAC C - 3'; WxR-F2887: 5'- TGG TGT TGT CCT TCT GTG GTC A -3' and WxR-R3831: 5'- CGT CGT CGT CGG GGT TCA GGT A -3'; WxR-F3509: 5'-GTC AGC CTC CCT CTC CCT TCT C -3' and WxR-R4701: 5'- TAC AGC CGT GGG AGA GGA GAT A -3'; WxR-F4451: 5'- AAG TCA AAT TAG CCA CGT AG -3' and WxR-R5625: 5'- CGG CTT GGC GTA CGT TGC AT -3'; WxR-F5447: 5'- CAC GCA CAC CCC AAA CAG AC -3' and WxR-R7899: 5'- CTT GGG TTA AAA TCT TGC GA -3'; WxR-F7675: 5'- AAC TGT TCT TGA TCA TCG CA -3' and WxR-R9867: 5'- ATT GTA TAC CGT TCC GTA TC -3'; WxR-F9709: 5'-AGG AGA AGT ATC CGG GCA AG -3' and WxR-R12465: 5'- TCT TGA CTT GTC CCG GAC CAT -3'; WxR-F14654: 5'- CTT ATA TTG TGG GAC GGA GA -3' and WxR-R15022: 5'- AGA AGC CTA ATA TAC CAC GA -3'; as well as WxR-F-284: 5'- ATG CAA TGA GAA GAT CTG TA -3' and WxR-R209: 5'- TCT TAT AAG CCG GAG TCT TG -3'. The resulting 12 fragments were sequenced using a BigDye™ Terminator Cycle Sequencing Ready Reaction Kit V3.0 (Applied Biosystem), an ABI PRISM® 377 DNA Sequencer and an ABI PRISM® 3100 Genetic Analyzer (both available from Applied Biosystem), along with the respective primers.

### Example 6

For efficient positive-negative selection, the hygromycin phosphotransferase gene (hph) was chosen as a positive selection marker, and the diphtheria toxin A chain gene (DT-A; Pappenheimer, AM Jr (1977) Diphtheria toxin. Annu Rev Biochem 46: 69-94) was chosen as a negative selection marker. DT-A is toxic to eukaryotic organisms including plants because it inhibits protein synthesis by catalyzing ADP-ribosylation of elongation factor (Yagi T, Ikawa Y, Yoshida K, Shigetani Y, Takeda N, Mabuchi I, Yamamoto T, Aizawa S (1990) Homologous recombination at c-fyn locus of mouse embryonic stem cells with use of diphtheria toxin A-fragment gene in negative selection. Proc. Natl. Acad. Sci. usa 87:9918-9922, Czako M and An G (1991) Expression of DNA coding for diphtheria toxin chain A is toxic to plant cells. Plant Physiol 95:687-692, Bellen HJ, D'evelyn D, Harvey M, Elledge SJ (1992) Isolation of temperature-sensitive diphtheria toxin in yeast and their effects on Drosophila Cells. Development 114 787-796). To examine the transformation frequency, pRIT and pINA134 were used as positive and negative control vectors, respectively. As in the case of pRW, pRIT and pINA134 also carried hph as a positive selection marker, and they used a rice actin 1 promoter and the first intron thereof to maximize the transformation efficiency.

Transformation with pRIT confirmed that 1500 independent callus were obtained from 400 mature seeds. pINA134 was also transformed in the same manner to study the effect of negative selection, confirming that transformation with pINA134 resulted in more than 99% lethality. PCR analysis suggested that each non-surviving callus had the DT-A gene. Most of surviving callus transformed with pINA134 were partially or completely deficient in the DT-A gene. These results suggest that the DT-A gene is effective for negative selection in rice when ligated to a highly active promoter. It is also suggested that two DT-A genes located apart from each other provide an extremely low probability of yielding recombinants deficient in both DT-A genes.

The Waxy targeting vector pRW was used to perform transformation. To confirm the efficiency of transformation and the effect of positive-negative selection, pRIT and pINA134 were also used for transformation and the number of resulting hygromycin-resistant callus was determined for each case. As shown in Table 1, the number of callus obtained was about 1500 for pRIT, about 60 for pINA134, and about 100 for pRW, averaged over six transformation experiments.

PCR was carried out using the pair of H1F and W2R primers to confirm homologous recombination at the Waxy locus. The H1F primer encodes the loxP sequence located at the 3'-end of the En/Spm sequence, whereas the W2R primer encodes any sequence outside of the homologous recombination region. When used against callus undergoing homologous recombination, these primers can confirm the amplification of a fragment of approximately 8 kb. A total of 630 surviving callus obtained from six experiments were analyzed with these primers to find six independent positive callus lines (A-I, A-II, B, C, E, F). As shown in Table 1, among callus obtained in each experiment, one hundredth of them, on average, were PCR-positive. In some rare cases, amplification of different size fragments was observed. This would be ascribed to irregular integration or integration with deletion of the DT-A gene.

DNA was isolated from green leaves of whole plants regenerated from the six PCR-positive lines (A-I, A-II, B, C, E, F), and then digested with HindIII, MunI or EcoRV for Southern analysis. The Waxy fragment, which had been 14.7 kb long before recombination, was changed into 6.3 kb and 12.1 kb fragments in the PCR-positive recombinants. This was because a HindIII site was inserted into the first intron by homologous recombination. The 6.3 kb fragment hybridized to the Waxy promoter region (pW4.1), whereas the 12.1 kb fragment hybridized to the coding regions of the hph gene (Hm0.9) and the Waxy gene (cWx2.7). Similar results could be confirmed by analyses with MunI and EcoRV. Also, it was estimated from the intensity of detected signals that homologous recombination occurred on only one allele in all recombinants.

All the homologous recombinants (lines A-I, A-II, B, C, E, F) could be regenerated into whole plants, which were capable of fertilization and seed production. Among them, line A-I was analyzed for segregation of the Waxy gene by iodine staining and PCR. Starch present in the pollen and seeds of Nipponbare having the Waxy gene is composed of amylose and amylopectin, and hence stained in dark blue by iodine staining. In contrast, starch present in glutinous rice having the waxy gene is composed of amylopectin only, and hence stained in reddish-brown. In line A-I, the ratio between pollen grains stained in dark blue and reddish-brown was 1:1. In addition, 13 of 53 seeds obtained from line A-I were stained in reddish-brown. The same experiment was performed on seeds of lines A-II, B, C, E and F, indicating that the staining ratio was almost the same as that observed in line A-I. These results suggest that homologous recombination occurred on only one allele of the Waxy gene in the whole plants regenerated from lines A-I, A-II, B, C, E and F. Subsequently, to distinguish the genotypes of the resulting seeds, the seeds were germinated and DNA was isolated from green leaves for PCR analysis. The primers H1F and W2R were used for confirmation of homologous recombination, while the primers Pro1 and Ex2 were used for detection of the original Waxy locus. Pro1 and Ex2 amplify a 1.3 kb fragment around the first intron. As a result of PCR analysis, there was complete agreement between phenotype by iodine staining and genotype by PCR analysis, and 27 of the seeds stained in dark blue had a heterozygous genotype. Namely, it was indicated that F₁ progenies segregated in a genotypic ratio of 1:2:1 and hence homologous recombination occurred on only one allele. Moreover, it was also indicated that the homologously recombined gene was correctly inherited to the next generation.

Since there would be a possibility that the Waxy gene would fail to function due to unexpected reconstruction of the genomic genes, Southern analysis was performed to confirm that the hph-En/Spm gene was correctly integrated into the Waxy first intron. For use in analysis, the genomic DNA was digested with HindIII, KpnI, MunI and EcoRV for the progeny of line A-I and with HindIII, MunI and EcoRV for the progeny of lines A-II, B, C, E and F. More than one whole plant was used for analysis in each case. These restriction enzymes were selected for the reasons that no polymorphism for these enzymes was found at the Waxy locus between Nipponbare and Shimokita and that these enzymes were also present in the hph-En/Spm gene. pW4.1, Hm0.9 and cWx2.7 were used as probes. As a result, all fragments of expected size were observed in F₁ progenies, indicating that homologous recombination occurred as expected and was correctly inherited to the progeny.

Among the progeny plants of lines A-I, B and C, those homozygous for the recombination region were used for nucleotide sequencing. The genomic DNA of the homozygous individuals was analyzed to determine the nucleotide sequence of the Waxy region expected to undergo homologous recombination, confirming that there were no mutations or errors, e.g., unexpected deletions and insertions.

Thus, the resulting rice plants were shown to have correct homologous recombination in view of Southern analysis of six transformants obtained from six independent experiments, PCR analysis of F1 progenies, phenotype and genotype of F1 progenies, segregation mode in F1 progenies by Southern analysis, as well as sequencing results of the recombination region in lines A-I, B and C. Moreover, this gene modification method was also shown to be reproducible.

**Table 1.**

| Targeting efficiency | | | | | |
|---|---|---|---|---|---|
| Experiment | Transformants ^{a} | Callus surviving negative selection ^{b} | Callus surviving positive-negative selection ^{c} | PCR-positive callus | Targeting frequency (× 10⁻⁴) |
| A | 2097 | 22 | 92 | 2 | 9.5 |
| B | 1550 | 80 | 153 | 1 | 6.5 |
| C | 1976 | 176 | 114 | 1 | 5.1 |
| D | 1159 | 10 | 61 | 0 | _^{d} |
| E | 589 | 0 | 100 | 1 | 17.0 |
| F | 1782 | 61 | 110 | 1 | 5.6 |
| Average | 1526 | 58 | 105 | 1 | 7.3 |

| | | | | | |
|---|---|---|---|---|---|
| a: the number of hygromycin-resistant callus obtained by transforming 1-5 gFW callus with pRIT, calculated as per weight of callus (about 40 gFW) used for transformation with pRW. | | | | | |
| b: the number of hygromycin-resistant callus obtained by transforming 1-5 gFW callus with pINA134, calculated as per weight of callus (about 40 gFW) used for transformation with pRW. | | | | | |
| c: the number of hygromycin-resistant callus obtained by transforming 40 gFW callus with pRW. | | | | | |
| d: < 8.6 × 10⁻⁴ | | | | | |

### References

In addition to the documents stated above, the following documents were used as references:
(1) Echt CS Schwartz D (1981) Evidence for the inclusion of controlling elements within the structural gene at the waxy locus in maize. Genetics 99:275-284;
(2) Hajdukiewicz P, Svab Z, Maliga P (1994) The small, versatile pPZP family of Agrobacterium binary vectors for plant transformation. Plant Mol Biol 25:989-994;
(3) McElroy D, Blowers AD, Jenes B, Wu R (1991) Construction of expression vectors based on the rice actin (Act1) 5' region for use in monocot transformation. Mol Gen Genet 231: 150-160;
(4) Nelson, OE (1962) The Waxy locus in maize. I. Intralocus recombination frequency estimates by pollen and by conventional analyses. Genetics 47:737-742;
(5) Wessler, SR, Varagona, MJ (1985) Molecular basis of mutations in the Waxy locus of maize: correlation with the fine Structure genetic map. Proc. Nail. Acad. Sci. usa 82:4177-4181;
(6) US Patent No. 6187994, Application No. 9765613;
(7) International Patent Public Disclosure WO 9925821, International Application No. WO 98US24610; and
(8) US Patent No. 5464764, Application No. 014083.

SEQUENCE LISTING

<110> Japan Tabacco Inc.

<120> Method for modifying higher plant genome

<130> 011248

<160> 7

<210> 1
<211> 12
<212> DNA
<213>

<400> 1
agctgggatc cc 12

<210> 2
<211> 30
<212> DNA
<213>

<400> 2
gtataatgta tgctatacga agttatgttt 30

<210> 3
<211> 30
<212> DNA

<213>

<400> 3
gtttggtcat attatgtact taagctaagt 30

<210> 4
<211> 20
<212> DNA
<213>

<400> 4
acacaaataa ctgcagcttc 20

<210> 5
<211> 28
<212> DNA
<213>

<400> 5
gagctgggac gtggtgagag ccgacatg 28

<210> 6
<211> 23
<212> DNA
<213>

<400> 6
gtaggtagac cgggggcaat gag 23

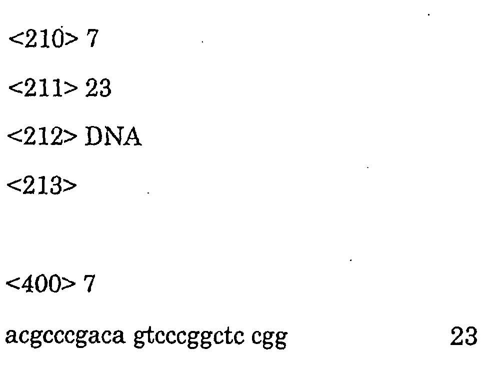

## Claims

1. A gene construct for modifying the genome of higher plants by homologous recombination, which contains the following elements between BR (right border sequence) and BL (left border sequence) of T-DNA in the following orientation from BR:
(1) a first negative selection marker gene in an expressible state;
(2) a first cloning site for incorporating the 5' region of a gene to be homologously recombined with a target gene in the host genome;
(3) a positive selection marker gene in an expressible state;
(4) a second cloning site for incorporating the 3' region of the gene to be homologously recombined with the target gene in the host genome; and
(5) a second negative selection marker gene in an expressible state, which may be the same as the first negative selection marker.

2. A gene construct of claim 1, which further includes (6) a transcription termination region, preferably a transcription termination region of En/Spm type transposon, in downstream of the first cloning site.

3. A gene construct of claim 1 or 2, which further includes (7) recombinase recognition sequences in upstream and downstream of the positive selection marker gene.

4. A gene construct of any one of claims 1-3, wherein one or both of the cloning sites for incorporating the 5' and 3' regions of the gene to be homologously recombined with the target gene in the host genome are multiple cloning sites.

5. A gene construct of any one of claims 1-4, wherein the first negative selection marker gene in an expressible state is located between a maize ubiquitin 1 promoter (pUbi) and a cauliflower mosaic virus 35S terminator (t35S).

6. A gene construct of any one of claims 1-5, wherein the positive selection marker gene is in an expressible state by being placed between a rice actin promoter (pAct) along with the 1st intron (iAct) of rice actin and a cauliflower mosaic virus 35S terminator (t35S).

7. A gene construct of any one of claims 1-6, wherein the second negative selection marker gene is in an expressible state by being placed between a cauliflower mosaic virus 35S promoter (p35S) along with the 1st intron of castor bean catalase and a cauliflower mosaic virus 35S terminator (t35S).

8. A gene construct of any one of claims 1-7, wherein the directions of transcription of the first and second negative selection marker genes are opposite to each other.

9. A gene construct of any one of claims 1-8, wherein the positive selection marker gene is selected from the group consisting of hygromycin resistance gene, kanamycin resistance gene, neomycin resistance gene, and herbicide resistance gene (for example, bar gene), while the negative selection marker gene is selected from the group consisting of diphtheria toxin protein A chain gene (DT-A), codA gene, cytochrome P-450 gene, and barnase gene.

10. A vector for plant transformation, which contains a gene construct of any one of claims 1-9.

11. A vector for plant transformation which contains a gene construct of any one of claims 1-9, wherein the 5' region of the gene to be homologously recombined with the target gene in the host genome is incorporated into the first cloning site, and the 3' region of the gene to be homologously recombined with the target gene in the host genome is incorporated into the second cloning site.

12. A method for producing a higher plant (preferably monocotyledon) having a modified genome by means of homologous recombination, comprising the steps of:
(a) introducing a vector for plant transformation of claim 11 into an Agrobacterium strain which contains Ti plasmid;
(b) allowing said Agrobacterium strain to infect a cell, tissue or callus of a plant;
(c) performing negative and positive selections to select a cell, tissue or callus undergoing homologous recombination;
(d) culturing the cell or tissue to form a callus in the case that selection was performed with a cell or tissue;
(e) culturing the callus in a regeneration medium to generate an individual plant which is heterozygous for homologous recombination in the genome; and
(f) fertilizing said plant to yield a gene-modified higher plant which is homozygous for homologous recombination.

13. A method of claim 12, which includes the step of deleting the positive selection marker gene and its promoter and terminator, as well as the transcription termination region, if present, from the region of homologous recombination in the genome of the plant cell, before or after the step (d), (e) or (f).

14. A method of claim 13, wherein the deletion step comprises the Cre/lox recombination system, the R/RS recombination system, or the FLP-FRT recombination system.
